# EUROPEAN PATENT SPECIFICATION

(11) **EP 1 250 160 B1**
(45) Date of publication and mention of the grant of the patent: **24.08.2005**
(21) Application number: 01902209.4
(22) Date of filing: 26.01.2001
(51) Int. Cl.: A61L 2/22, A01N 59/00, A01N 25/06

(54) **ANTIMICROBIAL FLASH-DRY DISINFECTANT AEROSOL**
ANTIMIKROBIELLES, FLASH-TROCKENDES DESINFEKTIONSAEROSOL
AEROSOL DESINFECTANT ANTIMICROBIEN A SECHAGE ECLAIR

(30) Priority: 26.01.2000 CA 2297002
(43) Date of publication of application: 23.10.2002
(73) Proprietor: Triosyn Holding Inc., Mirabel, Québec J7J 1M3 (CA)
(72) Inventor: MESSIER, Pierre, Jean, St-Sauveur, Québec J0R 1R7 (CA); ST-LOUIS, Jean-Pierre, Prévost, Québec J0R 1T0 (CA); TANELLI, Joe, St-Léonard, Québec H1R 1S6 (CA); BOURGET, Stéphane, Montréal, Québec H2P 2K3 (CA)
(74) Representative: Beresford, Keith Denis Lewis
(86) International application number: PCT/CA2001/000102
(87) International publication number: WO 2001/054740

(56) References cited:
- EP-A- 0 099 209
- EP-A- 0 132 038
- EP-A- 0 404 015
- EP-A- 0 508 525
- US-A- 4 678 658
- US-A- 4 716 032
- US-A- 5 916 568

## Description

The present invention relates to an aerosol apparatus (e.g. pressurised) for dispensing an antimicrobial agent in an aerosol spray onto a surface to be disinfected. The aerosol apparatus contains a flash-dry disinfectant composition comprising said antimicrobial agent (e.g. the antimicrobial agent may be hydrogen peroxide).

Disinfectant compositions are known which comprise an alcohol(s) and hydrogen peroxide, compositions which have a bactericidal effect when applied to a surface (please see U.S. patent nos 5,916,568 and 5,868,998 which describe the use of hydrogen peroxide as a bactericide.

It would be advantageous to have a means for facilitating the application of or dispensing of a disinfectant composition onto a surface so as to facilitate the antimicrobial effect of such disinfectant composition while at the same time being able to facilitate or accelerate the drying of the surface to be disinfected.

The present invention provides, in combination, an aerosol spray apparatus for applying an anti-microbial agent to a surface to be disinfected and a liquid flash-dry disinfectant composition, in accordance with claim 1. The invention also provides a method for disinfecting a surface and a liquid flash-dry composition for disinfecting a surface. Preferred embodiments of the invention are defined in the dependent claims.

In accordance with the present invention the components of the flash-dry disinfectant composition (i.e. the antimicrobial agent and the flash vaporisation component) may be mixed together just prior to the flash-dry disinfectant composition being applied to a surface.

Accordingly said apparatus may comprise a first container means containing said antimicrobial agent and a second container means containing said flash vaporisation component wherein said aerosol spray apparatus comprises mixing means for mixing said antimicrobial agent and said flash vaporisation component together prior to dispensing said liquid flash-dry disinfectant composition through said spray nozzle.

In accordance with the present invention the antimicrobial agent may. For example, be hydrogen peroxide; however any other suitable (known) antimicrobial agent able to be incorporated into a formulation able to be disposed on a surface by means of an aerosol spray may be used.

The flash vaporisation component may, for example, comprise an alkanol of formula ROH wherein R is a group containing from 1 to 6 carbon atoms such as for example ethanol, isopropanol, butanol and the like. Additionally the flash vaporisation component may, for example, comprise a ketone of up to six carbon atoms (e.g.acetone); an ether of up to six carbon atoms; a halogenated organic compound such as, for example, freon, chloroform; etc..

In accordance with the present invention a flash-dry disinfectant composition (in a container means or as being sprayed as an aerosol onto the surface to be disinfected) comprises 10 to 30% by volume of hydrogen peroxide, 10 to 85% by volume of a flash vaporisation component for example an alcohol such as ethanol. The flash-dry disinfectant composition comprises a remainder component or element which may, for example, comprise or be water.

In accordance with the present invention a flash-dry disinfectant composition (in a container means or as being sprayed as an aerosol onto the surface to be disinfected) may for example in particular comprise 10 to 30% by volume of hydrogen peroxide, 10 to 85 % by volume of an alcohol such as for example ethanol and 10 to 65 % by volume of water.

More particularly, the flash-dry disinfectant composition of the present invention may comprise any (liquid) flash vaporisation component which is able to impart to the composition a flash vaporisation characteristic i.e. to achieve in a relatively, (desired or necessary) short period of time after applying the flash dry aerosol to a surface, a state wherein the surface is essentially dry leaving behind the antimicrobial agent (i.e. a surface has achieved a desired or necessary state of dryness). The flash dry liquid may for example be one which is able to evaporate at a relatively rapid rate at ambient room temperature and pressure, i.e. having a high volatility (e.g at 15 degrees centigrade or higher such as at 20 to 25 degrees centigrade). The flash-dry disinfectant composition may for example, comprise a flash vaporisation component comprising one or more of the above mentioned materials; it may for example comprise an alkanol comprising from 2 to 5 carbon atoms (e.g ethanol, isopropanol, butanol etc...), an alkoxy substituted alkane (i.e. an ether) comprising from 2 to 4 carbon atoms (e.g. diethyl ether - ethoxy ethane, ....); etc... The flash dry materials may be obtained from any known suppliers. An alcohol for example may be obtained from any known supplier such as Fisher Scientific, Signa-Aldrich (e.g. as a composition comprising 82-95% by volume ethanol, 2 to 10% by volume water and 3 to 10% by volume methanol).

The antimicrobial agent may as mentioned above be hydrogen peroxide; it may also be a compound that forms peroxide in an aqueous medium (i.e. in situ). The hydrogen peroxide may be obtained from any known supplier such as Fisher Scientific, Signa-Aldrich (e.g. as a composition comprising 30% by volume hydrogen peroxide and 70% by volume water).

The aerosol apparatus may take any desired (known) form provided that it may deliver the flash-dry disinfectant composition to a surface in aerosol (e.g. in micro aerosol).

The aerosol apparatus may for example comprise a pressurised container containing a predosed amount of inert propellant gas component in addition to the flash-dry disinfectant composition. In this case, the basic aerosol container is configured to be able to contain or store the flash-dry disinfectant composition under pressure which is due to the additional presence in the container of an inert pressurising gas component such as air, nitrogen gas, etc.. Alternatively the basic aerosol container may comprise there within or otherwise be associated with a secondary pressurized gas container which is appropriately coupled in any suitable (known) manner to the spray nozzle so as to be able to induce the disinfectant composition through the nozzle as an aerosol spray. The propellant gas may be any gas which is at least essentially inert with respect to the flash-dry disinfectant composition components (e.g. nitrogen gas, air, and the like); it may also be a freon gas.

The aerosol apparatus alternatively may comprise a mechanism for manually introducing atmospheric air into the container so as to generate a positive pressure therein as mentioned above; an example of this latter type of aerosol apparatus is described in U.S. patent no. 5,265,775.

The aerosol apparatus may for example be configured in any known manner so as to be able to generate or provide a spray having droplet sizes of for example from 1 to 100 microns; larger sized droplets may of course be used provided that the desired flash dry effect is achieved when the composition is applied to a surface to be disinfected.

The aerosol container may have any type of known spray nozzle system coupled to the pressurised container for dispensing an aerosol spray or jet onto a surface. The nozzle is of course configured so as to be able to pass from a normal closed configuration (no spray being developed) to an active open configuration, the opened configuration being such so as to allow the internal positive pressure persisting in the aerosol container to urge the flash-dry disinfectant composition out of the container as an aerosol spray. Suitable (aerosol) type may be obtained from Electron Microscopy Sciences, Pennsylvania USA (i.e. Spray Safe Atomizer (64186); from Four Star Chemicals, ,California USA (i.e Aluminum and epoxy coated cans); and from CEODEUX Firetec, out of Luxembourg (i.e. 3-Six and nine liter extinguisher type sprayer).

In the drawings which illustrate example embodiments of the present invention :
- Figure 1: is a schematic illustration of a known type of aerosol can containing a flash dry disinfection composition of the present invention;
- Figure 2: is a schematic illustration of an alternative type of aerosol can containing separated liquid components of a flash dry disinfection composition of the present invention; and
- Figure 3: is a is a schematic illustration of an additional type of (known) aerosol can containing separated liquid components of a flash dry disinfection composition of the present invention.

With respect to the drawings the same reference numerals will be used to designate the same element(s).

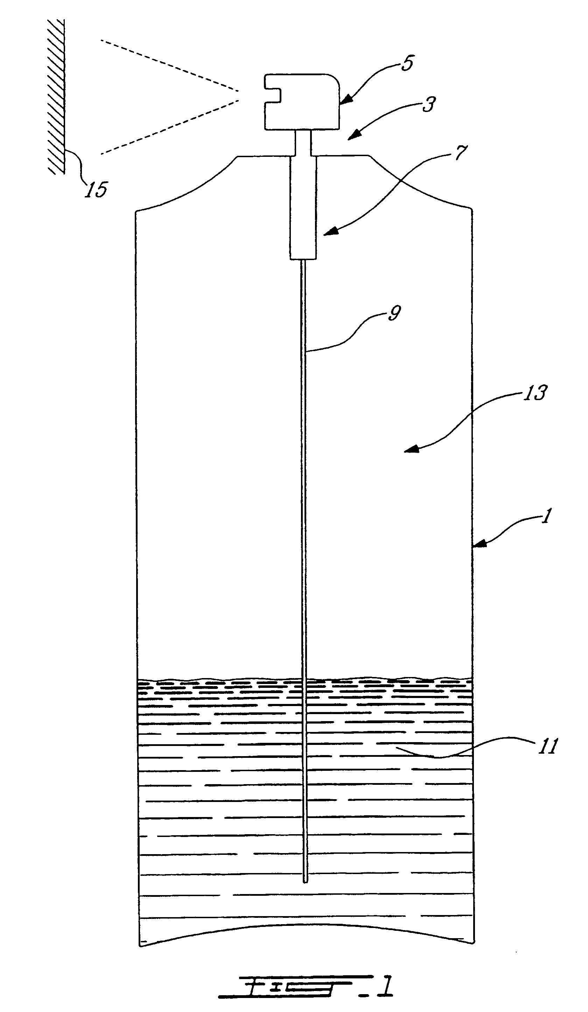

Referring to figure 1 the aerosol spray can comprises an outer cylindrical container shell 1, a spray valve nozzle assembly 3 (comprising a spray nozzle 5) and a siphoning assembly 7 (including a siphoning tube 9). The spray can also contains therein a liquid flash-dry disinfection composition 11 in accordance with the present invention. A pressure space 13 is located above the disinfection composition 11 and may contain a pressurised gas for inducing the disinfection composition to pass up through the siphoning assembly tube 9 to the spray nozzle 5 for ultimately being passed therethrough as a spray aerosol indicated in dotted outline; the spray of course being directed to the surface 15 to be sterilized.

In accordance with the embodiment illustrated in figure 1 the various liquid component forming the disinfectant composition may be poured into the container shell 1; the valve nozzle assembly 3 is then used to cap or seal (in a permanent or removable manner) the container shell 11 in gas or air tight fashion; and then the container is pressurized (through the nozzle assembly 3 - nozzle 5 removed) using an inert gas to a pressure ranging for example from 414 to 1380 kPa (60 psig to 200 psig). Alternatively, instead of an inert gas the container once the nozzle assembly is in place may be pressurized by being filled with a suitable (known) low boiling point refrigerant gas such as a freon.

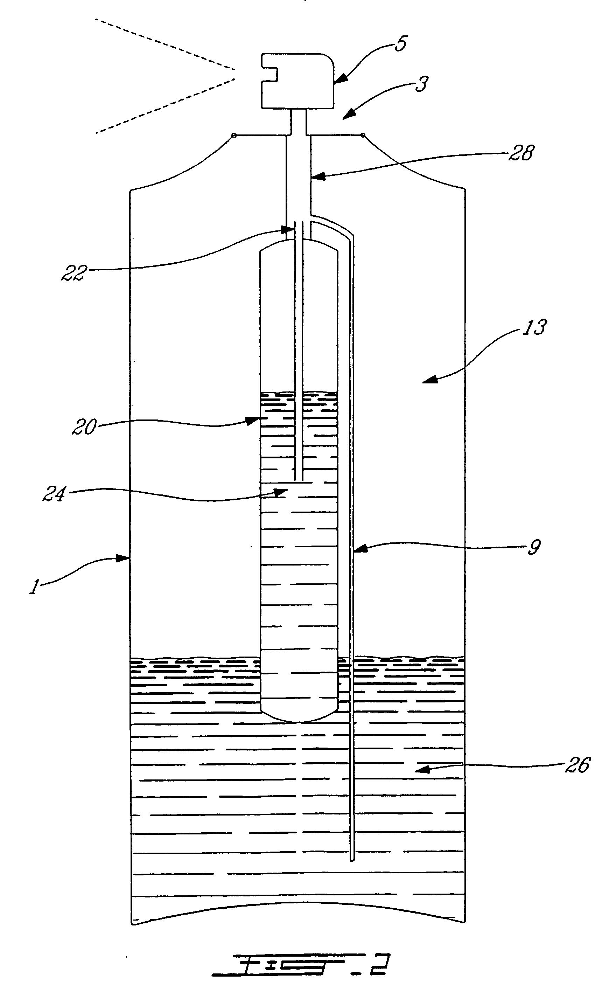

Turning to figure 2; this figure illustrates an acrosol spray can assembly wherein components of the flash-dry disinfectant composition are kept apart in separate container means until the time at which they are to be sprayed. This can assembly also comprises an outer cylindrical container shell 1, a spray valve nozzle assembly 3 (comprising a spray nozzle 5) and a siphoning tube 9. The spray can however, also has an inner container 20 which is also provided with a siphon tube assembly indicated generally by the reference numeral 22. The inner container 20 contains a first liquid component 24 of the flash-dry disinfection composition whereas a second liquid component (indicated by the reference numeral 26) is located outside the inner container 20 but inside the container shell 1. The can assembly is also provided with a siphoning and mixing assembly 28. A pressure space 13 is located above the second disinfection component 26 and may contain a pressurised gas for inducing the first and second liquid components 24 and 26 to pass up through respective siphoning assembly tubes 9 and 22 to the mixing assembly 28 and then on to the spray nozzle 5 for ultimately being passed therethrough as a spray aerosol indicated in dotted outline. The system shown may be pressurised through the nozzle assembly 3 (first removing the nozzle 5). In this case when nozzle 5 is depressed both liquid components 24 and 26 will be forced into the mixing assembly, mixed and then sprayed out the nozzle 5.

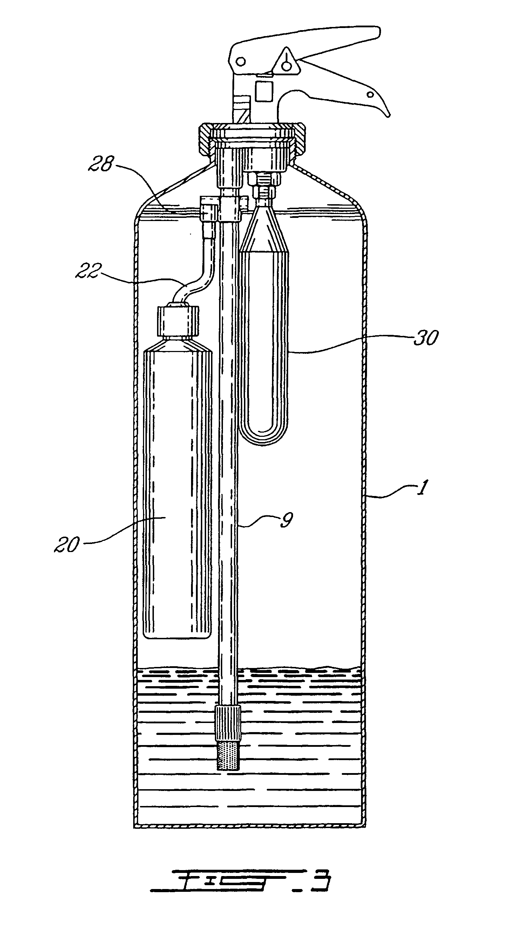

Referring to figure 3, this figure illustrates an alternate aerosol spray can assembly wherein components of the flash-dry disinfectant composition are kept apart in separate container means until the time at which they are to be sprayed; the spray container may be obtained from CEODEUX Firetec, out of Luxembourg. This assembly differs from that shown in figure 2 in that the pressurization is accomplished by use of a scaled pressurized CO₂ canister 30. In this case when the respective nozzle is initially depressed the carbon dioxide in the canister 30 is allowed to escape (in known fashion) so as to induce CO₂ pressurization of the system including that of the second liquid component. When the nozzle is depressed again the two liquid components are forced to mix together in the mixing assembly the resultant flash-dry disinfectant composition is expelled out through the spray nozzle as a spray aerosol.

The various elements or components of the present invention such as the materials for the flash-dry disinfectant composition (including their proportions in the flash-dry disinfection composition), the components or element of the aerosol apparatus (i.e. the spray nozzle head) are of course to be selected with a view to the desired or necessary ends herein i.e. disinfection/drying.

The surface(s) to be treated in accordance with the present invention may be hard (e.g. metal) or soft (e.g. carpet)

The following examples illustrate various example flash-dry disinfectant compositions of the present invention. The various microorganisms used for the tests as well as the procedure (protocol 4) used to effect various tests will be discussed after the examples.

### Example 1

### EVAPORATION TIME FOR VARIOUS AEROSOL STERILIZER

| **Solutions** | **Evaporation Time** | | | | | | |
|---|---|---|---|---|---|---|---|
| | 1 minute | 2 minutes | 5 minutes | 15 minutes | 30 minutes | 40 minutes | 45 minutes |
| H₂O | wet | wet | humid | droplets | droplets | droplets | dry |
| H₂O₂ 10% | wet | wet | humid | driplets | droplets | droplets | dry |
| H₂O₂ 10% in ETOH | wet | humid | dry | dry | dry | dry | dry |
| ETOH 95% | humid | dry | dry | dry | dry | dry | dry |

### Example 2

The sterilizing effect of an aerosol containing H₂O₂/ETOH on various materials inoculated with BG for 60 minutes contact time.
Temperature : 20.7°C

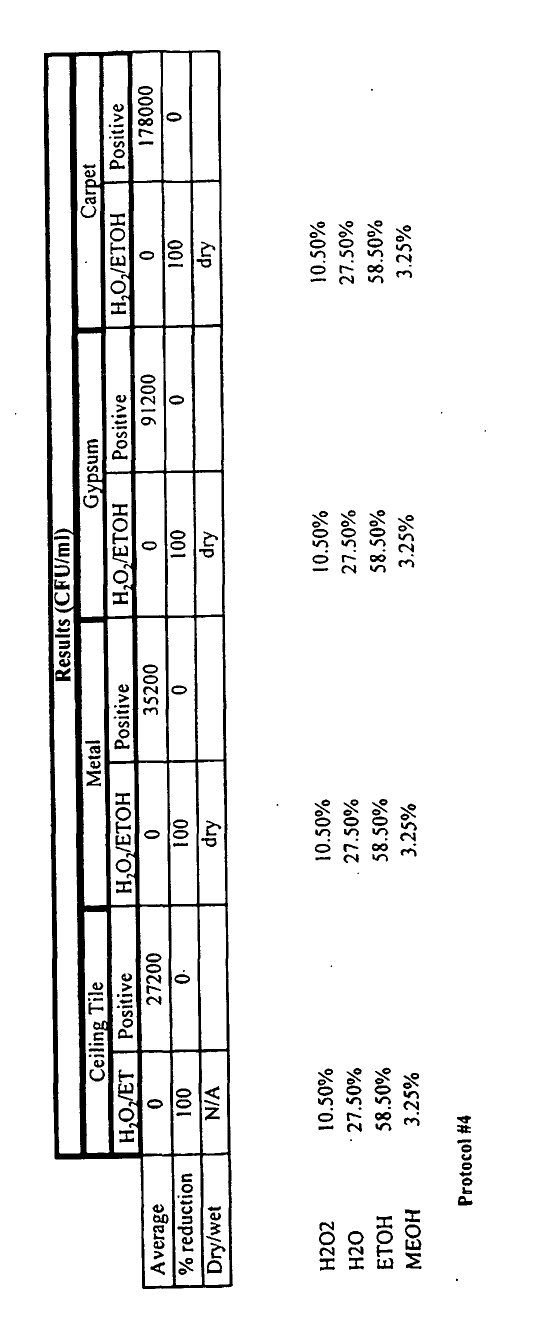

### Example 3

The comparative performance of a sterilizing agent against MS2 phage on various surfaces for 60 minutes contact time.

Temperature: 22.4°C

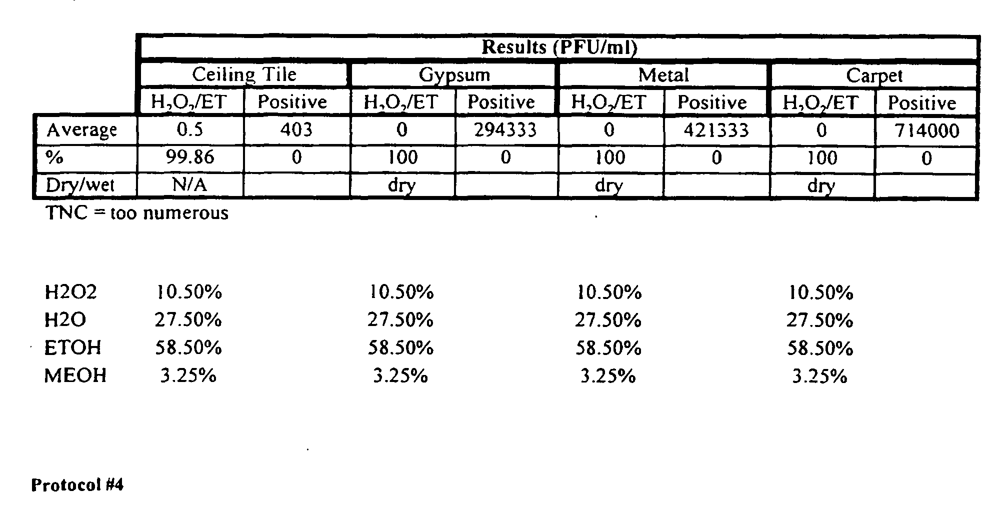

### Example 4

Sterilizing effect of H₂O₂ 10% in Ethanol (lot 991208) on Gypsum wallboard pieces inoculated with BG spores.

Temperature: 19.6°C

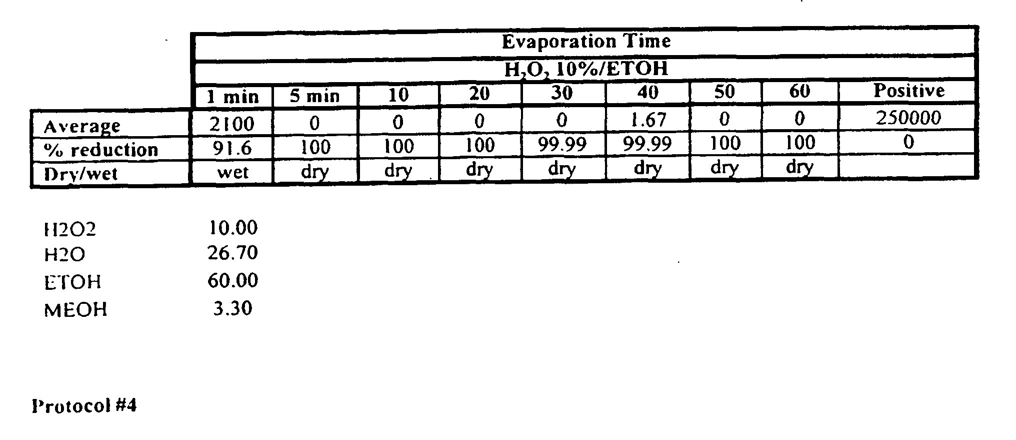

### Example 5

Performance of H₂O₂ in ETOH against BG spores on gypsum board places for 60 minutes contact time.

Temperature: 19.6°C

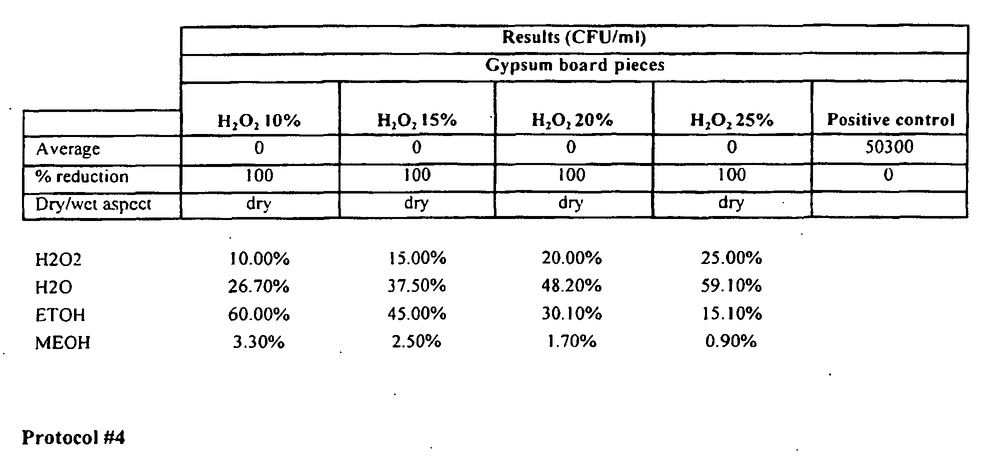

### Example 6

Performance of H₂O₂ 10% in methanol against BG spores on gypsum board pieces for different contact time

Temperature: 19.0°C

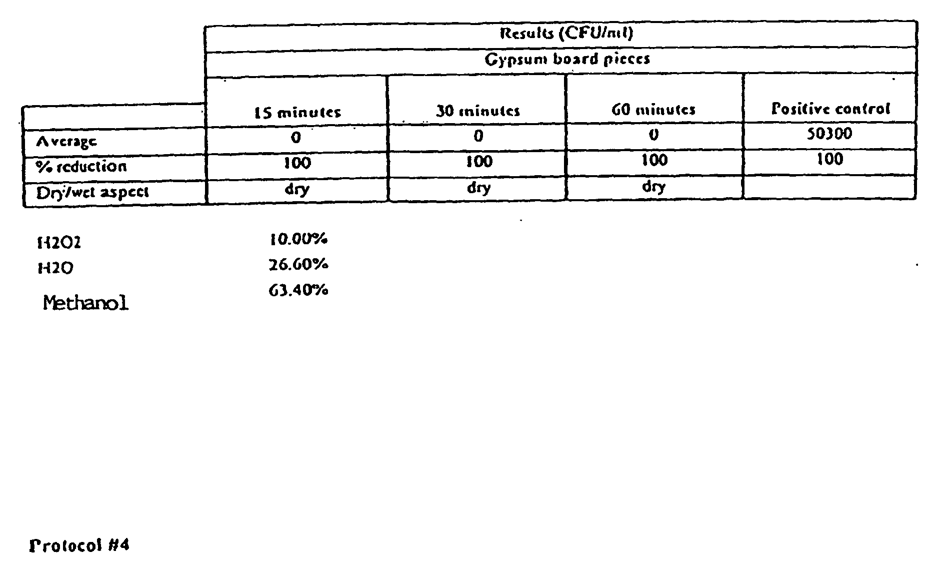

### Example 7

Performance of H₂O₂ 10% in Isopropyl alcohol against BG spores on gypsum board pieces for different contact time

Temperature: 19.0°C

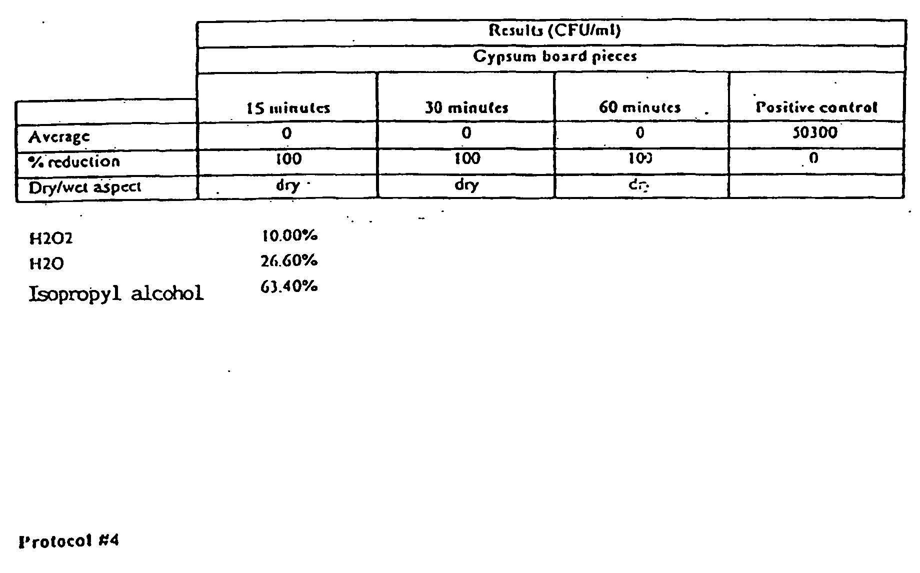

### Example 8

Performance of H₂O₂ 10% in propanol against BG spores on gypsum board pieces for different contact time

Temperature: 19.8°C

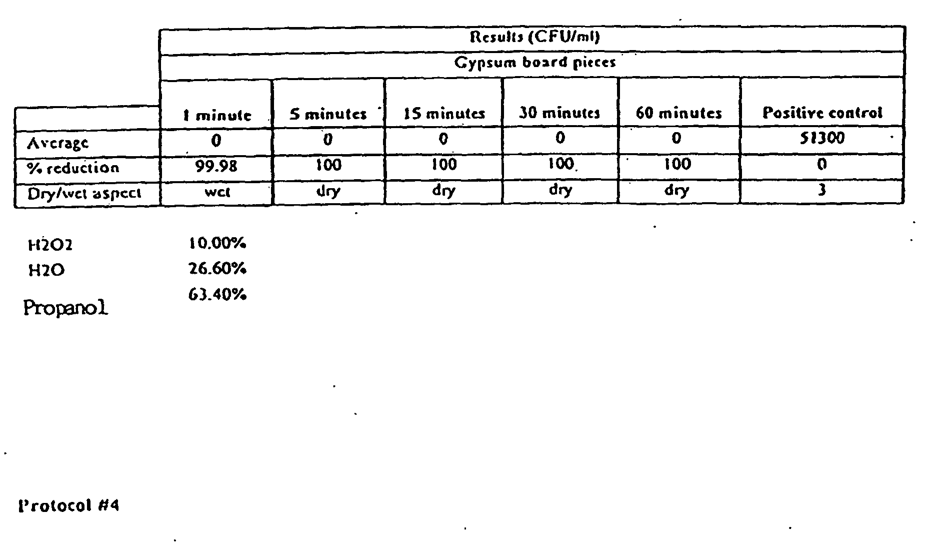

### Example 9

Performance of H₂O₂ 10% in isopropyl alcohol against BG spores on gypsum board pieces for different contact time

Temperature: 19.8°C

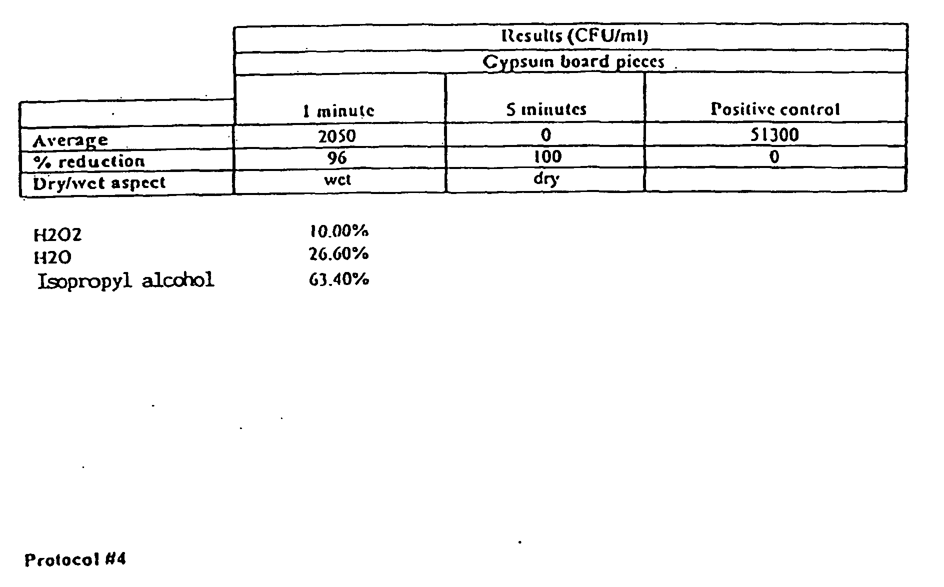

### Example 10

Performance of H₂O₂ 10% in methanol against BG spores on gypsum board pieces for different contact time

Temperature: 19.8°C

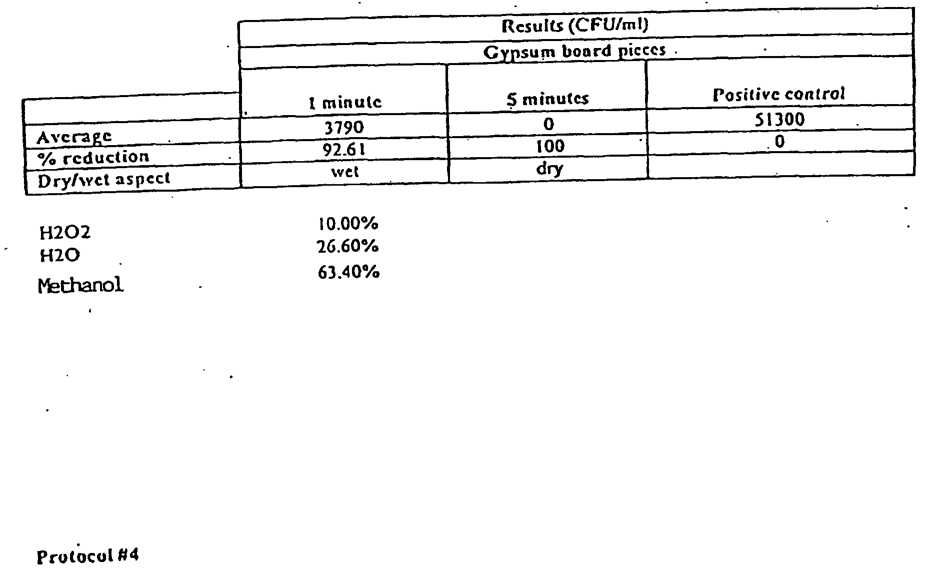

### Bacillus subtilis var niger spores (DG)

*Bacillus subtilis* var niger spores (ATCC.9372) is used as challenge organism. BG is a Gram-positive spore-forming bacteria that is widely used as an accepted surrogate for spore-forming bacterial BW agents such as *Bacillus anthracis*. The extreme protection that sporulation gives to this mircroorganism makes it one of the most difficult to eliminate. BG spores are produced following our internal Work Instructions based on standard microbiology procedures.

### MS2 coliphage

The other challenge organism used for the sterilizing tests is MS2 (ATCC 15597-B1). MS2 is an approximately 26 nm icosahedral bacteriophage that infects Escherichia coli (ATCC 15597). It is an accepted surrogate for viral BW agents, including Ebola and Venezuelan Equine Encephalitis. MS2 is prepared according to Dugway Proving Ground SOP.

### Protocol #4

Apparatus and Reagents
Gypsum wallboard piece of 10cm*10cm
Carpet piece of 10cm*10cm
Sheet metal piece of 10cm*10cm
Ceiling tile piece of 10cm*10cm
Tissue of partition panels piece of 10cm*10cm
NDS buffer
Incubator
*Bacillus subtilis* var niger spores and MS2 phage
Spray can (SureShot Atomizer, Model B sprayer, Milwaukee Sprayer MFG. CO. INC.,
Milwaukee WI, USA) with fin spray nozzle (#B30-040) filled with biocidal solutions and air or gaseous nitrogen.

### Procedure #4

1. With a sponge, spread the inoculum at a concentration of 10⁷ CFU/ml or PFU/ml over the clean surface of the pieces to be tested. Keep 1 cm border free of microorganisms.
2. Using the spray can, treat all the surface of the pieces for 3 to 5 seconds.
3. Wait for the chosen contact time.
4. Sample the experimental and positive control pieces of the gypsum board, sheet metal and ceiling tile with a swab in 9 ml of NDS buffer. Sample the experimental and positive control pieces of the carpet and tissue, by soaking the pieces in 27 ml of NDS buffer and then sampling 1 ml of the residual liquid.
5. For BG spores, place 0.2 ml of each dilution on TSA media; for MS2 phage, place 1.0 ml of each dilution on MS2 media. Incubate at 35°C for 24 hours.

### Results and discussion

Numerous experiments were perfonned to evaluate the comparative performance of TAS against MS2 phage and BG spores under different environmental conditions.

The Neutralizing Dilution Solution (NDS buffer) used for purpose of sampling contains sodium thiosulfate, which is the agent responsible for the neutralization of the oxidants (such as hydrogen peroxide) present in the experimental samples. The neutralization action of sodium thiosulfate prevents the oxidizing process from going on once the sampling has occurred, ensuring the accuracy of the chosen contact time.

The sterilizing solution is applied by means of an aerosol spray can (Sure Shot Atomizer, Model B sprayer, Milwaukee Sprayer MFG. CO. INC., Milwaukee, WI, USA) filled with the biocidal solution and air or gaseous nitrogen. The.gas propellant ensures the propulsion in the form of an aerosol of the sterilizing solution to be expelled. Adapted spray nozzles come in different sizes. We used the finest nozzle available (#B30-040) in order to produce an aerosol with droplet size as small as possible. The physico/chemical properties of the biocidal agents combined with the aerosol application method contribute to generate a flash-dry sterilizing solution.

Microbiological results are presented in the Examples 1 to 10. Prior experimentation proved the concentration of 10% of hydrogen peroxide in combination with ethanol (ETOH) to provide a 100% sterilizing efficacy as well as a short evaporation time. The performance of hydrogen peroxide 10% (H₂O₂ 10%) in combination with different alcohols against BG spores on gypsum board pieces was evaluated for various contact time throughout Examples 6 to 10. Positive results were observed for all the alcohols tested (isopropyl alcohol, methanol and propanol all in a concentration of 63,4%) and all the configurations with 5-minute contact time and above presented a 100% sterilizing efficacy. After 5 minutes of contact time all sample surfaces were left dry. It is worth noting that after I minute of contact time, an average of 96,1% of microorganisms are already eliminated.

The sterilizing efficacy of the hydrogen peroxide/ethanol solution was tested on various surfaces in order to assess its efficacy on different materials Examples 2 and 3. The solution was challenged with MS2 and BG for 60 minutes of contact time. We obtained reduction rates of a 100% for all the samples with the exception of the ceiling tile sample in Example 3 that yielded a 99,86% result.

The selected sterilizing solution (H₂O₂ 10% in ethanol) is a flash-dry biocidal solution with high and fast kill rate. The said solution combines a 100% sterilizing efficacy against MS2 and BG spores within 5 minutes of contact time with flash-dry properties.

## Claims

1. An aerosol spray apparatus for applying an anti-microbial agent to a surface to be disinfected, comprising:
an aerosol spray nozzle (5) coupled to a container apparatus (1) for containing a liquid disinfectant composition (11) able to be sprayed from the apparatus, and a liquid flash-dry disinfectant composition in the container apparatus, said flash-dry disinfectant composition being able, once sprayed in aerosol form onto the surface to be disinfected, to flash vaporize to leave an essentially dry surface having the anti-microbial agent deposited thereon;
**characterised in that**:
said liquid flash-dry disinfectant composition comprises: 10% to 30% by volume of hydrogen peroxide;
10% to 85% by volume of a flash vaporization component; and
a remainder component.

2. Apparatus as claimed in claim 1 wherein the flash vaporization component comprises an alcohol.

3. Apparatus as claimed in claim 1 wherein the flash vaporization component comprises an alkanol of formula ROH wherein R is a group containing 1 to 6 carbon atoms.

4. Apparatus as claimed in any preceding claim wherein the flash vaporization component comprises ethanol.

5. Apparatus as claimed in claim 1 wherein the flash-dry disinfectant composition comprises:
10% to 30% by volume hydrogen peroxide;
10% to 85% by volume of an alcohol; and
10% to 65% by volume water.

6. Apparatus as claimed in claim 5 wherein the alcohol is ethanol.

7. Apparatus as claimed in any preceding claim wherein the container apparatus comprises a first container for containing a gas propellant and a second container for containing the liquid disinfectant composition.

8. Apparatus as claimed in any of claims 1-7 wherein the container apparatus comprises a first container for containing a first liquid component and a second container for containing a second liquid component.

9. Apparatus as claimed in claim 8 wherein the container apparatus further comprises a third container for containing a gas propellant.

10. A liquid flash-dry disinfectant composition comprising a flash vaporization component for disinfecting a surface, said flash-dry disinfectant composition being able, once sprayed in aerosol form onto the surface to be disinfected, to flash vaporize to leave an essentially dry surface having an anti-microbial agent deposited thereon;
**characterised in that**:
said liquid flash-dry disinfectant composition comprises:
10% to 30% by volume of hydrogen peroxide;
10% to 85% by volume of a flash vaporization component; and
a remainder component.

11. A composition as claimed in claim 10 wherein the flash vaporization component comprises an alcohol.

12. A composition as claimed in claim 10 wherein the flash vaporization component comprises an alkanol of formula ROH wherein R is a group containing 1 to 6 carbon atoms.

13. A composition as claimed in any of claims 10 to 12 wherein the flash vaporization component comprises ethanol.

14. A composition as claimed in claim 10, comprising:
10% to 30% by volume hydrogen peroxide;
10% to 85% by volume of an alcohol, and
10% to 65% by volume water.

15. A composition as claimed in claim 14 wherein the alcohol is ethanol.

16. A method for disinfecting a surface, comprising:
applying by aerosol spray to a surface to be disinfected a liquid flash-dry disinfectant composition comprising a flash vaporization component which is able, once the flash-dry disinfectant composition is sprayed in aerosol form onto said surface, to flash vaporize to leave an essentially dry surface having an anti- microbial agent deposited thereon;
**characterised in that** said liquid flash-dry disinfectant composition is a composition as claimed in any one of claims 10 to 15.

## Patentansprüche

1. Aerosolsprühvorrichtung zum Anwenden eines anti-mikrobiellen Mittels auf eine zu desinfizierende Oberfläche, umfassend
eine Aeorsolsprühdüse (5), die mit einer Behältervorrichtung (1) für eine von der Vorrichtung sprühbare flüssige Desinfektionsverbindung (11) verbunden ist, und eine flüssige schnell-trocknende Desinfektionsverbindung in der Behältervorrichtung, wobei die schnell-trocknende Desinfektionsverbindung, sobald sie in Aerosolform auf die zu desinfizierende Oberfläche gesprüht wird, in der Lage ist, schnell zu verdunsten und so eine im wesentlichen trockene Oberfläche mit dem darauf aufgetragenen anti-mikrobiellen Mittel zu hinterlassen,
**gekennzeichnet dadurch, daß**
die flüssige schnell-trocknende Desinfektionsverbindung umfaßt:
10 bis 40 Vol-% Wasserstoffperoxid,
10 bis 85 Vol-% einer Schnellverdunstungskomponente, und eine Restkomponente.

2. Vorrichtung nach Anspruch 1, wobei die Schnellverdunstungskomponente einen Alkohol umfaßt.

3. Vorrichtung nach Anspruch 1, wobei die Schnellverdunstungskomponente ein Alkanol der Formel ROH umfaßt, wobei R eine 1 bis 6 Kohlenstoffatome enthaltende Gruppe ist.

4. Vorrichtung nach einem der vorhergehenden Ansprüche, wobei die Schnellverdunstungskomponente Ethanol umfaßt.

5. Vorrichtung nach Anspruch 1, wobei die schnell-trocknende Desinfektionsverbindung umfaßt:
10 bis 30 Vol-% Wasserstoffperoxid,
10 bis 85 Vol-% eines Alkohols, und
10 bis 65 Vol-% Wasser.

6. Vorrichtung nach Anspruch 5, wobei der Alkohol Ethanol ist.

7. Vorrichtung nach einem der vorhergehenden Ansprüche, wobei die Behältervorrichtung einen ersten Behälter für ein Treibgas und einen zweiten Behälter für die flüssige Desinfektionsverbindung umfaßt.

8. Vorrichtung nach einem der Ansprüche 1 bis 7, wobei die Behältervorrichtung einen ersten Container für eine erste flüssige Komponente und einen zweiten Behälter für eine zweite flüssige Komponente umfaßt.

9. Vorrichtung nach Anspruch 8, wobei die Behältervorrichtung ferner einen dritten Behälter für ein Treibgas umfaßt.

10. Flüssige schnell-trocknende Desinfektionsverbindung umfassend eine Schnellverdunstungskomponente zum Desinfizieren einer Oberfläche, wobei die schnell-trocknende Desinfektionsverbindung, sobald sie in Aerosolform auf die zu desinfizierende Oberfläche gesprüht wird, dazu in der Lage ist, schnell zu verdunsten und so eine im wesentlichen trockene Oberfläche mit einem darauf aufgetragenen anti-mikrobiellen Mittel zu hinterlassen,
**gekennzeichnet dadurch, daß**
die flüssige schnell-trocknende Desinfektionsverbindung umfaßt:
10 bis 30 Vol-% Wasserstoffperoxid,
10 bis 85 Vol-% einer Schnellverdunstungskomponente, und eine Restkomponente.

11. Verbindung nach Anspruch 10, wobei die Schnellverdunstungskomponente einen Alkohol umfaßt.

12. Verbindung nach Anspruch 10, wobei die Schnellverdunstungskomponente ein Alkanol der Formel ROH umfaßt, wobei R eine 1 bis 6 Kohlenstoffatome enthaltende Gruppe ist.

13. Verbindung nach einem der Ansprüche 10 bis 12, wobei die Schnellverdunstungskomponente Ethanol umfaßt.

14. Verbindung nach Anspruch 10, umfassend:
10 bis 30 Vol-% Wasserstoffperoxid,
10 bis 85 Vol-% eines Alkohols, und
10 bis 65 Vol-% Wasser.

15. Verbindung nach Anspruch 14, wobei der Alkohol Ethanol ist.

16. Verfahren zum Desinfizieren einer Oberfläche, umfassend:
Anwenden einer flüssigen schnell-trocknenden Desinfektionsverbindung auf eine zu desinfizierende Oberfläche mittels Aerosolsprühens, wobei die Verbindung eine Schnellverdunstungskomponente umfaßt, die, sobald die schnell-trocknende Desinfektionsverbindung in Aerosolform auf die Oberfläche gesprüht wird, in der Lage ist, schnell zu verdunsten und so eine im wesentlichen trockene Oberfläche mit einem darauf aufgetragenen anti-mikrobiellen Mittel zu hinterlassen,
**gekennzeichnet dadurch, daß** die flüssige schnell-trocknende Desinfektionsverbindung eine Verbindung nach einem der Ansprüche 10 bis 15 ist.

## Revendications

1. Un dispositif de pulvérisation d'aérosol pour l'application d'un agent anti-microbien sur une surface à désinfecter, comprenant :
- une buse de pulvérisation d'aérosol (5) couplée à un appareil conteneur (1) pour contenir une composition liquide désinfectante (11) apte à être pulvérisée du dispositif, et dans l'appareil conteneur une composition désinfectante liquide à séchage immédiat, ladite composition désinfectante à séchage immédiat étant apte, une fois pulvérisée sous forme d'aérosol sur la surface à désinfecter, à s'évaporer immédiatement pour laisser une surface sensiblement sèche sur laquelle est déposé l'agent anti-microbien ;
**caractérisé en ce que** ladite composition désinfectante liquide à séchage immédiat comprend :
- 10 % à 30 % en volume de peroxyde d'hydrogène ;
- 10 % à 85 % en volume d'un composant à vaporisation immédiate ; et
- un autre composant.

2. Le dispositif selon la revendication 1, dans lequel le composant à vaporisation immédiate comprend un alcool.

3. Le dispositif selon la revendication 1, dans lequel le composant à vaporisation immédiate comprend un alcanol de formule ROH où R est un groupe comportant 1 à 6 atomes de carbone.

4. Le dispositif selon l'une quelconque des revendications précédentes, dans lequel le composant à vaporisation immédiate comprend l'éthanol.

5. Le dispositif selon la revendication 1, dans lequel la composition désinfectante à séchage immédiat comprend :
10 % à 30 % en volume d'un peroxyde d'hydrogène ;
10 % à 85 % en volume d'un alcool ; et
10 % à 65 % en volume d'eau.

6. Le dispositif selon la revendication 5, dans lequel l'alcool est l'éthanol.

7. Le dispositif selon l'une quelconque des revendications précédentes dans lequel l'appareil conteneur comprend un premier réservoir pour contenir un gaz propulseur et un second réservoir pour contenir la composition désinfectante liquide.

8. Le dispositif selon l'une quelconque des revendications 1 à 7, dans lequel l'appareil conteneur comprend un premier réservoir pour contenir un premier composant liquide et un second réservoir pour contenir un second composant liquide.

9. Le dispositif selon la revendication 8, dans lequel l'appareil conteneur comprend en outre un troisième réservoir pour contenir un gaz propulseur.

10. Une composition désinfectante liquide à séchage immédiat comprenant un composant à vaporisation immédiate pour la désinfection d'une surface, ladite composition désinfectante à séchage immédiat étant apte, une fois pulvérisée sous forme d'aérosol sur la surface à désinfecter, à s'évaporer instantanément pour laisser une surface sensiblement sèche sur laquelle est déposé un agent anti-microbien, **caractérisée en ce que** :
- ladite composition désinfectante liquide à évaporation immédiate comprend :
- 10 % à 30 % en volume de peroxyde d'hydrogène ;
- 10 % à 85 % en volume d'un composant à vaporisation immédiate ; et
- un autre composant.

11. Une composition selon la revendication 10, dans laquelle le composant à vaporisation immédiate comprend un alcool.

12. Une composition selon la revendication 10, dans laquelle le composant à vaporisation immédiate comprend un alcanol de formule ROH où R est un groupe contenant 1 à 6 atomes de carbone.

13. Une composition selon l'une quelconque des revendications 10 à 12 dans laquelle le composant à vaporisation immédiate comprend l'éthanol.

14. Une composition selon la revendication 10, comprenant :
- 10 % à 30 % en volume de peroxyde d'hydrogène ;
- 10 % à 85 % en volume d'un alcool; et
- 10 % à 65 % en volume d'eau.

15. Une composition selon la revendication 14, dans laquelle l'alcool est l'éthanol.

16. Un procédé pour désinfecter une surface, comprenant :
l'application par pulvérisation d'aérosol à une surface à désinfecter d'une composition désinfectante liquide à séchage immédiat comprenant un composant à vaporisation immédiate qui est apte, une fois que la composition désinfectante à séchage immédiate a été pulvérisée sous forme d'aérosol sur ladite surface, à s'évaporer immédiatement pour laisser une surface sensiblement sèche sur laquelle est déposé un agent anti-microbien ;
**caractérisée en ce que** ladite composition désinfectante liquide à séchage immédiat est une composition selon l'une quelconque des revendications 10 à 15.
